# EUROPEAN PATENT APPLICATION

(11) **EP 1 681 063 A1**
(43) Date of publication of application: **19.07.2006**
(21) Application number: 04771663.4
(22) Date of filing: 13.08.2004
(51) Int. Cl.: A61K 38/18, A61P 9/00, A61P 25/00, A61P 25/28

(54) **AGENT FOR IMPROVING MENTAL DISORDERS**

(30) Priority: 14.10.2003 JP 2003354411
(71) Applicant: Takeo, Satoshi, Kitatsuru-gun, Yamanashi 4090126 (JP); Kringle Pharma Inc., Osaka 560-0082 (JP)
(72) Inventor: TAKEO, Satoshi, Kitatsuru-gun, Yamanashi 4090126 (JP); NAKAMURA, Toshikazu, Kyoto-shi, Kyoto 6068333 (JP); TAKAGI, Keiko, Hino-shi, Tokyo 192-0024 (JP); TAKAGI, Norio, Hino-shi, Tokyo 192-0024 (JP)
(74) Representative: Findeisen, Marco
(86) International application number: PCT/JP2004/011696
(87) International publication number: WO 2005/034985

(57) **Abstract**

The present invention relates to an agent for improving a mental disorder due to cerebral dysfunction, an agent for inhibiting vascular hyperpermeability, a composition for improving a mental disorder due to cerebral dysfunction, a composition for inhibiting vascular hyperpermeability, a method of improving a mental disorder due to cerebral dysfunction, a method of inhibiting vascular hyperpermeability, and the use of a hepatocyte growth factor in producing a pharmaceutical preparation.

## Description

### Technical Field

The present invention relates to an agent for improving a mental disorder due to decline in brain functions occurred in association with cerebrovascular disorders. More particulary, the present invention relates to a pharmaceutical preparation useful in improving mental disorders due to cerebral dysfunction occurred in association with cerebrovascular disorders and neurodegenerative disorders, more specifically decline in learning and memory function, and inhibition of dementia and recovery therefrom, which comprises hepatocyte growth factor as an active ingredient. The present invention also relates to a pharmaceutical preparation for inhibiting vascular hyperpermeability such as cerebrovascular hyperpermeability.

### Background Art

Hepatocyte growth factor (hereinafter also referred to as HGF) was initially identified as a growth factor for mature hepatocytes, and its gene (cDNA) was cloned in 1989 (see Biochemical and Biophysical Research Communications, 1984, Vol. 122, pp. 1450-1459, and Nature, 1989, Vol. 342, pp. 440-443.

Up to now, HGF has been revealed to exhibit various biological activities such as growth promotion, cellular migration, morphogenesis induction and apotosis prevention in various types of cells including hepatocytes (see The Journal of Cell Biology, 1985, Vo. 129, pp. 1177-1185; The Journal of Biochemistry, 1986, Vol. 119,pp.591-600; International Review of Cytology, 1999, Vol. 186, pp. 225-260; and Kidney International, 2001, Vol. 59, pp. 2023-2038).

The biological activities of HGF are exhibited via its receptor c-Met tyrosine kinase, and HGF functions in repairing and protecting various kinds of injured tissues, via the diverse biological activities.

As one of tissue regenerating and protecting functions of HGF, a neovascularization promoting activity can be mentioned. HGF not only promotes growth and migration of vascular endothelial cells but also has a strong neovascularization inducing activity in vivo (see The Journal of Cell Biology, 1992, Vol. 119, pp. 629-641; Proceedings of the National Academy of Sciences of the United States of America, 1993, Vol. 90, pp. 1937-1941; Circulation, 1998, Vol. 97, pp. 381-390; and Hypertension, 1999, Vol. 33, pp. 1379-1384).

Furthermore, HGF has an activity of inhibiting apotosis of vascular endothelial cells (see, for example, Journal of Hypertension, 2000, Vol. 18, pp. 1411-1420; Hypertension, 2001, Vol. 37, pp. 581-586; and Diabetes, 2002, Vol. 51, pp. 2604-2611).

Substances and methods for improving mental disorders due to cerebral dysfunction such as learning and memory dysfunction and dementia have been studied and investigated in many fields, and the results have been increasingly accumulated. According to the results, the conventional studies on improving mental disorders due to cerebral dysfunction is roughly divided into a method of improving the brain energy metabolism of activating cellular activities by allowing brain cells to absorb nutrients effectively, and a method of improving circulation in the brain by improving blood circulation in the brain to sufficiently supply nutrients and oxygen necessary for brain cells, and therapeutic methods or drugs having the respective pathological actions have been studied. Mental disorders attributable to dementia are divided into and recognized as 2 types, that is, Alzheimer type dementia caused by neurodegenerative disorders and cerebrovascular dementia caused by cerebrovascular disorders, and drugs or therapeutic methods corresponding thereto have been studied.

With respect to the relationship between HGF and brain disorders, survival of cultured cerebral neurons is promoted in vitro by HGF, and expression of HGF mRNA and c-met mRNA in the brain is significantly increased in the survival areas having brain disorders, and on the basis of these findings, it is described that HGF is useful in prevention and therapy of the central nervous disorders (see JP-A No. 08-89869). In these descriptions, the in vitro suppression of number of dead cells in the cultured hippocampal neurons is simply demonstrated, and expression of HGF mRNA and c-met mRNA in the damaged brain is merely shown, and a description of the protective effect of HGF on the functions of the hippocampal neurons or the damaged brain is not determined.

HGF exhibits a neurotrophic activity on hippocampus, cerebral cortex, dopaminergic midbrain, cerebellar granules, sense, motoneuron and various neurons (nerve cells) (see Brain Research. Molecular Brain Research, 1995, Vol. 32, pp. 197-210; The Journal of Biochemistry, 1986, Vol. 119, pp. 591-600; The Journal of Neuroscience Research, 1996, Vol. 43, pp. 554-564; Neuron, 1996, Vol. 17, pp. 1157-1172; The Journal of Neuroscience, 2000, Vol. 20, pp. 326-337; and The European Journal of Neuroscience, 1999, Vol. 11, pp. 4139-4144).

Furthermore, HGF is reported to reduce cerebral infarct size in ischemic rats and decreased the number of vessels in the infarct areas (see Neurological Research, 2001, Vol. 23, pp. 417-423).

However, these literatures report necrosis of nerve cells after ischemia and do not examine mental disorders due to cerebral dysfunction such as memory and learning dysfunction.

Furthermore, there is no report showing the relationship between HGF and vascular hyperpermeability such as cerebrovascular hyperpermeability caused by the disruption of the blood-brain barrier, etc.

### Disclosure of the Invention

An object of the present invention is to provide an agent for improving mental disorder due to cerebral dysfunction. Another object of the present invention is to provide an agent for inhibiting vascular hyperpermeability caused by the disruption of the blood-brain barrier, etc.

The present inventors have made extensive efforts to solve the problem described above, and as a result, they have found that HGF inhibits cerebral apotosis in a cerebral embolism-induced animal model, and ameliorates decline in memory and learning function in the cerebral embolism-induced animal model. Furthermore, the present inventors have found that HGF inhibits cerebrovascular hyperpermeability caused by the disruption of the blood-brain barrier, etc. in a cerebral embolism-induced animal model. On the basis of these findings, the present invention has been completed. That is, the present invention relates to:
(1) an agent for improving a mental disorder due to cerebral dysfunction, comprising a hepatocyte growth factor;
(2) the agent according to the above (1), wherein the mental disorder is a decline in learning function;
(3) the agent according to the above (1), wherein the mental disorder is a decline in memory function;
(4) the agent according to the above (1), wherein the mental disorder is dementia;
(5) an agent for inhibiting vascular hyperpermeability, comprising a hepatocyte growth factor;
(6) the inhibiting agent according to the above (5), wherein the vascular hyperpermeability is cerebrovascular hyperpermeability;
(7) a composition for improving a mental disorder due to cerebral dysfunction, comprising a hepatocyte growth factor and a pharmaceutically acceptable additive;
(8) the composition according to the above (7), wherein the mental disorder is a decline in learning function;
(9) the composition according to the above (7), wherein the mental disorder is a decline in memory function;
(10) the composition according to the above (7), wherein the mental disorder is dementia;
(11) a composition for inhibiting vascular hyperpermeability, comprising a hepatocyte growth factor and a pharmaceutically acceptable additive;
(12) the composition according to the above (11), wherein the vascular hyperpermeability is cerebrovascular hyperpermeability;
(13) a method of improving a mental disorder due to cerebral dysfunction, which comprises administering a hepatocyte growth factor to a mammal;
(14) the method according to the above (13), wherein the mental disorder is a decline in learning function;
(15) the method according to the above (13), wherein the mental disorder is a decline in memory function;
(16) the method according to the above (13), wherein the mental disorder is dementia;
(17) a method of inhibiting vascular hyperpermeability, which comprises administering a hepatocyte growth factor to a mammal;
(18) the method according to the above (17), wherein the vascular hyperpermeability is cerebrovascular hyperpermeability;
(19) use of a hepatocyte growth factor in producing a pharmaceutical preparation for inhibiting a mental disorder due to cerebral dysfunction;
(20) the use according to the above (19), wherein the mental disorder is a decline in learning function;
(21) the use according to the above (19), wherein the mental disorder is a decline in memory function;
(22) the use according to the above (19), wherein the mental disorder is dementia;
(23) use of a hepatocyte growth factor in producing a pharmaceutical preparation for inhibiting vascular hyperpermeability; and
(24) the use according to the above (23), wherein the vascular hyperpermeability is cerebrovascular hyperpermeability.

Furthermore, the present invention encompasses a gene therapy of mental disorders due to cerebral dysfunction or vascular hyperpermeability, which comprises not only administering HGF but also introducing an HGF gene.

The agent for improving mental disorders due to cerebral dysfunction according to the invention ameliorates mental disorders due to cerebral dysfunction occurred in association with cerebrovascular disorders (for example, brain infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.) and neurodegenerative disorders (for example, multiple sclerosis, Parkinson's disease, Parkinson's syndrome, Huntington's chorea, cerebrovascular dementia and Alzheimer dementia), epilepsy, head injury, etc.

The agent for inhibiting vascular hyperpermeability according to the invention inhibits blood hyperpermeability caused by the disruption of the blood-brain barrier etc. in the brain based on cerebrovascular disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.) and blood leakage, cerebral edema, subcutaneous hemorrhage and bleeding tendency due to vascular hyperpermeability in various tissues (including internal organs).

The agent for inhibiting vascular hyperpermeability according to the present invention can further suppress the disruption of the blood-brain barrier, etc. , so that inclusion of unnecessary substances and toxic substances in the brain can be prevented, and thus brain tumors and senile plaques (plaques generated by deposition of β-amyloid protein in a part of the brain) induced by the toxic substance can also be prevented.

### Brief Description of the Drawing

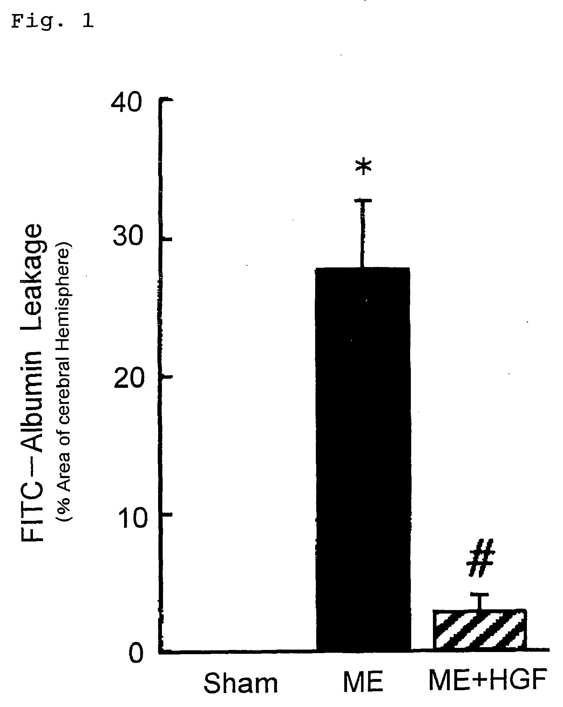

Fig. 1 is a graph showing the effect of HGF on cerebrovascular hyperpermeability attributable to microsphere embolism in the rat brain.

### Best Mode for Carrying Out the Invention

The mental disorders in the present invention refer to disorders in mental functions, such as memory and learning function, insight, language and judgment, due to cerebral dysfunction occurred in association with cerebral disorders, such as blood circulation disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.) and neurodegenerative disorders (for example, multiple sclerosis, Parkinson's disease, Parkinson's syndrome, Huntington's chorea, cerebrovascular dementia and Alzheimer dementia), epilepsy, head injury, etc.

The vascular hyperpermeability in the present invention means leakage of plasma components and other blood components in various tissues (for example, brain, skin, internal organs) and refers to, for example, leakage of plasma components and other blood components in the brain due to blood circulation disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.).

HGF used in the present present invention is a known substance, and HGF prepared by various methods can be used insofar as it is purified to such an extent that it can be used as a pharmaceutical preparation. In the method of producing HGF, for example, a primary culture cell or an established cell line producing HGF is cultured, and the HGF can be obtained by separation and purification from its culture supernatant, etc. Alternatively, a gene encoding HGF is integrated by genetic engineering techniques into a suitable vector, which is then transformed into a suitable host, and an objective recombinant HGF can be obtained from the culture supernatant of the transformant (see, for example, Nature, 1989, Vol. 342, pp 440-443, JP-ANo. 5-111382, Biochem. Biophys. Res. Commun. 163, 967 (1989), etc.). The cell host is not particularly limited, and various kinds of host cells can be used conventionally in genetic engineering techniques, for example, *Escherichia coli,* yeasts or animal cells. Insofar as HGF obtained in this manner has substantially the same action as that of wild HGF, one or more (for example, several) amino acid(s) in the amino acid sequence thereof may be substituted, deleted and/or added, and its sugar chain may also be similarly substituted, deleted and/or added.

The agent for improving mental disorders and the agent for inhibiting vascular hyperpermeability according to the present invention are used in improving mental disorders attributable to brain disorders not only in humans but also in other mammals (for example, bovine, horse, porcine, sheep, canine, feline, etc.) or leakage of plasma components and other blood components in the brain or other tissues.

The agent for improving mental disorders and the agent for inhibiting vascular hyperpermeability according to the present invention may take various pharmaceutical forms such as a liquid preparation, a solid preparation, a capsule, etc., and may be generally formed into an injection, an inhalation, a suppository or an oral preparation comprising HGF only or together with conventional carriers. The injection can be prepared according to the known method, for example by dissolving HGF in a suitable solvent such as sterile purified water, physiological saline or a buffer solution containing glucose and other auxiliary agents, then filtering it with a filter or the like for sterilization and filling the filtrate into an aseptic container. Suitable auxiliary solubilizers such as alcohols (ethanol, etc.), polyalcohols (propylene glycol, polyethylene glycol, etc.) and nonionic surfactants (polysorbate 80, polyethylene hardened castor oil 50, etc.) may also be used in combination. Sesame oil, soybean oil or the like is used as an oily solution, and auxiliary solubilizers such as benzyl benzoate, benzyl alcohol, etc. may be simultaneously used. The prepared injection is usually filled into a suitable ampoule. The content of HGF in the injection is regulated in the range of generally about 0.0002 to 0.2 w/v%, preferably about 0.001 to 0.1 w/v%. The liquid preparation such as an injection is desirably preserved in a frozen state or after removal of water by lyophilization or the like. For use, the lyophilized preparation is re-dissolved at use in distilled water for injection.

The oral preparation is prepared in the form of tablets (including sugar-coated tablets, film-coated tablets and enteric-coated tablets), granules, fine particles, powders, capsules (including soft capsules and enteric-coated capsules), liquids, emulsions and syrups. These preparations are produced by the methods known in the art, and carriers used ordinarily in the field of pharmaceutical formulation, for example, lactic acid, starch, sucrose and magnesium stearate are used. The suppository can also be prepared in a conventional manner using common formulation means with use of a customary base material (for example, cacao butter, laurin butter, glycerogelatin, macrogol, Witepsol, etc.). The inhalation can also be prepared according to the conventional means in pharmaceutical formulation. The content of HGF in the preparation can be suitably adjusted depending on the dosage form and the disease to be applied.

HGF used in the present invention, together with a biodegradable polymer, can be formulated into a sustained-release preparation (including sustained-release microcapsules). By formulating HGF particularly into a sustained-release preparation, effects such as maintenance of blood concentration, reduction in administration frequency, and mitigation of side effects can be expected. The sustained-release preparation can be produced according to the known methods described in, for example, Drug Delivery System, Chapter 3 (published by CMC, 1986), etc. The biodegradable polymer used in the sustained-release preparation can be suitably selected from the known biodegradable polymers, and examples thereof include polysaccharides such as starch, dextran and chitosan; proteins such as collagen and gelatin; polyamino acids such as polyglutamic acid, polylysine, polyleucine, polyalanine and polymethionine; polylactic acid; polyglycolic acid; lactic acid/glycolic acid polymer or copolymer; polycaprolactone; poly-β-hydroxybutyric acid; polymalic acid; polyacid anhydride; polyester such as fumaric acid/polyethylene/vinylpyrrolidone copolymer; polyorthoester; polyalkyl cyanoacrylic acid such as polymethyl-α-cyanoacrylic acid; and polycarbonate such as polyethylene carbonate and polypropylene carbonate. The biodegradable polymer is preferably polyester, more preferably a polylactic acid or a lactic acid/glycolic acid polymer or copolymer. When the polylacic acid/glycolic acid polymer or copolymer is used, the composition ratio (lactic acid/glycolic acid) (mol%) varies depending on the period of sustained release, and for example, when the intended period of sustained release is about 2 weeks to about 3 months, preferably about 2 weeks to about 1 month, the composition ratio is about 100/0 to about 50/50. The weight-average molecular weight of the polylactic acid/polyglycolic acid polymer or copolymer is generally about 5,000 to about 20,000. The polylactic acid/glycolic acid copolymer can be produced according to the known production method described in, for example, JP-A No. 61-28521. The produced sustained-release preparation is preserved under aseptic conditions until operation, and after craniotomy, the sustained-release preparation is used by keeping it in a suitable site in the brain (for example, in the case of cerebral infarction, several sites around the infarction) under aseptic conditions. The compounding ratio of the biodegradable polymer to HGF in the sustained-release preparation is not particularly limited, and for example, HGF is about 0.01 w/w% to 30 w/w% relative to the biodegradable polymer.

The agent for improving mental disorders and the agent for inhibiting vascular hyperpermeability according to the present invention can be administered via a suitable administration route depending on the form of the pharmaceutical preparation. For example, the agent for improving mental disorders and the agent for inhibiting vascular hyperpermeability according to the present invention can be administered in the form of an injection intravenously, intraarterially, subcutaneously, intramuscularly or intracerebrally. The dose is controlled suitably depending on the symptom, age, weigh, etc. of a patient, and the dose of HGF is usually about 0.001 mg to 1000 mg, preferably about 0.01 mg to 100 mg, which is administered preferably once or several times per day.

In recent years, gene therapy using an HGF gene has been reported (see Circulation, 96, No. 3459 (1997); Nature Medicine, 5, 226-230 (1999); Circulation, 100, No. 1672 (1999); and Gene Therapy, 7, 417-427 (2000)) and has become technically established. The present invention encompasses administration of not only the above-mentioned HGF, but also an agent for mental disorders and vascular hyperpermeability comprising HGF gene introduction. Hereinafter, gene therapy with HGF is described.

The "HGF gene" used in the present invention refers to a gene capable of expressing HGF. Specifically, the HGF gene includes a gene prepared by integrating HGF cDNA described in Nature, 1989, Vol. 342, pp. 440-443, Japanese Patent No. 2777678, Biochem. Biophys. Res. Commun., 163, 967(1989), Biochem. Biophys. Res. Commun., 172, 321 (1990) etc., into a suitable expression vector (non-virus vector, virus vector). Nucleotide sequences of cDNA encoding HGF are not only described in the literatures described above, but are also registered in databases such as GenBank. Accordingly, a suitable DNA portion based on the information on these sequences is used as a primer for PCR, and subjected to RT-PCR reaction, for example, with mRNA from the liver, whereby the HGF cDNA can be cloned. Such cloning can be easily carried out by those skilled in the art according to basic books such as Molecular Cloning 2nd Edition, Cold Spring Harbor Laboratory Press (1989).

The HGF gene of the present invention is not limited to that described above, and any gene can be used as the HGF gene in the present invention so far as it can express a protein having substantially the action similar to that of HGF. Among DNAs hybridizing under stringent conditions with the above cDNA or DNAs encoding a protein comprising an amino acid sequence wherein one or more (preferably several) amino acid(s) is/are substituted in, deleted from and/or added to the amino acid sequence of the protein encoded by the above cDNA, any DNA encoding a protein having the action of HGF falls under the scope of the HGF gene of the present invention. The DNA can be easily obtained, for example, by the conventional hybridization method or PCR method, specifically referring to basic books such as Molecular Cloning supra.

The HGF gene of the present invention, similar to the above-described HGF protein, can be applied to amelioration of mental disorders and inhibition of vascular hyperpermeability.

When a gene therapeutic agent containing the HGF gene as an active ingredient is administered to a patient, it can be administered in a usual manner, for example, according to methods described in Fundamental Techniques of Gene Therapy, Special Issue of Experimental Medicines, Yodosha Co., Ltd., 1996; Gene Introduction & Expression Analysis Experimental Methods, Special Issue of Experimental Medicines, Yodosha Co. , Ltd., 1997, and Handbook of Development and Study of Gene Therapy edited by the Japan Society of Gene Therapy and published by N.T.S., 1999.

The form of the pharmaceutical preparation can take various known forms adapted to each administration mode described above.

The content of the DNA in the pharmaceutical preparation can be regulated suitably depending on the disease to be treated, the age, weight, etc. of patients, and usually the content of the DNA of the present invention is about 0.0001 to 100 mg, preferably about 0.001 to 10 mg.

The HGF gene and HGF can be used independently, or the two can also be used in combination.

### Examples

Hereinafter, the present invention is described in more detail by reference to the Examples and Formulation Examples, but the present invention is not limited thereto. Experimental Example 1. Effect of HGF on the decline in memory, learning functiones caused by cerebral embolism with microspheres

### 1. Experimental animal

Wistar male rats each weighing 220 to 250 g were used as an experimental animal. The animals were freely given feed and water and acclimated in an artificial environment at a constant temperature (23±1°C), constant humidity (55±5%) and illumination for a predetermined time (a cycle of 12 hours of light and 12 hours of darkness) according to the National Institute of Health Guide for the Care and Use of Laboratory Animals, and the Guideline for Experimental Animal Care, issued by the Prime Minister's Office of Japan.

### 2. Sample

Human recombinant DNA of hepatocyte growth factor (HGF) was prepared according to the known method (Nakamura et al., Nature 342:440-443, 1989) and used as a test sample.

### 3. Experimental method

### (1) Preparation of a model with microsphere-induced cerebral embolism

A model with microsphere-induced cerebral embolism was produced by the method described previously (Stroke 24, 415-420 (1993), Br. J. Pharmacol., 118, 33-40 (1996), J. Biol. Chem. , 277, 6382-6390 (2002)) with some modification. Rats were anesthetized by intraperitoneal administration of 40 mg/kg sodium pentobarbital (Nembutal, Abbott Laboratories, Canada, USA), and then fixed in a supine position. After midline incision, the right common carotid artery was removed, and the blood flow in right external carotid artery and right pterygopalatine artery were temporarily ligated. 700 microspheres (diameter of 48 µm, NEN-005, New England Nuclear Inc., Boston, USA)) suspended in 20 w/v% dextran solution was injected into right common carotid artery via a needle (25 G, TERUMO, Tokyo, Japan) connected to a polyethylene catheter (3 French size, Atom Medical Co., Ltd., Tokyo, Japan). After injection, the puncture wound was repaired with a surgical glue (Aron Alpha A, Sankyo Co., Ltd., Tokyo, Japan), and blood flow of the right external carotid artery and right pterygopalatine artery was recirculated, and the surgical site was sutured. It took about 2 minutes to occlude the blood flow of the right external carotid and right pterygopalatine arteries. The same operation as described above was carried out, and the rats to which 20 w/v% dextran solution only was injected was used as sham operation (Sham) rats.

The rats having cerebral embolism were divided at random into an HGF administered group and a solvent (vehicle) administered group. The continuous injection of HGF into the cerebral ventricle was conducted by administering a solution of HGF in physiological saline continuously for 7 days into the right cerebral ventricle via an osmotic pressure pump (Alzet model 2001, Alzet, CA, USA) from 10 minutes or 15 hours after injection of the microspheres, whereby 10 to 30 µg HGF was administered per animal.

### (2) Test of spatial memory and learning function using Morris water maze.

A test of spatial memory and learning function was started on day 12 after the cerebral embolism described in the above (1) when the rats were judged to be capable of swimming without neurologic deficits after cerebral embolism. A round pool having a diameter of 170 cm and a height of 45 cm was used as a water maze. Water (23±1°C) was introduced to a height of 30 cm into the pool and the illumination around the pool was kept unchanged.

The pool was divided into 4 quadrants, and in one of the quadrants, a transparent acryl resin platform (diameter 12 cm) was placed 1.5 cm below the surface of the water. The quadrant in the opposite side on a horizontal axis to the platform was assigned a first quadrant, and the other quadrants were assigned second, third and fourth quadrants respectively in the clockwise direction. That is, the platform was arranged in the predetermined position in the third quadrant. A geometrical pattern serving as a visual index was arranged around the pool, and this arrangement was kept constant during the experiment.

The rat was quietly released on water towards the internal wall of the pool. As the place where the rat was released on water, any one of the three quadrants other than the quadrant having the platform was selected at random.

A CCD camera (AXIS 60, Cosmicar TV lens, diameter of 4.8 mm, Artist, Japan) was installed just above the pool (the distance between the lens and the bottom of the pool was 2.10 m), and an image signal of rat's swimming track was monitored. This image signal was incorporated into a computer, and the time to climb onto the platform (escape latency), and the whole swimming track, were analyzed by analysis software (TARGET/2, Neuroscience, 9801, NEC, Japan). The swimming time was 180 seconds at the maximum, and when the rat could not climb onto the platform within 180 seconds, the rat was forced to being transferred to the platform, kept there for 30 seconds and then taken out from the pool. The above procedure was regarded as 1 trial.

In the test of spatial memory and learning function, 3 trials (= 1 session) were carried out per day at 1-hour intervals between the trials. The test was carried out for 3 consecutive days (that is, on days 12 to 14 after the cerebral embolism) and 9 times in total, and the place where each rat was released on water was changed at random among the sessions and among the trials.

For the purpose of examining whether or not memory of the past was kept, a retention test was carried out on days 21 and 28 after the cerebral embolism. In the retention test, the same swimming procedure as on day 14 after the cerebral embolism, that is, on the third day of the test of spatial memory and learning function, was carried out.

### (4) Results

Tables 1 to 3 show the results of escape latency in the water maze test used for investigating the spatial memory and learning function of the rats.

Table 1 shows the spatial memory and learning function on days 12, 13 and 14 days after the operation. In the sham operation group, the escape latency is shortened as the number of trials was increased, indicating that the rats had memory, learning abilities. The escape latency in the microsphere-induced cerebral embolism group was hardly shortened in the second and third trials on day 12 after the operation and even in the trials on the next day and on two days later, while the escape latency in the HGF administered group was shortened in a dose-dependent manner.

In the table, Sham is the sham operation group, ME is the microsphere-induced cerebral embolism group, ME + HGF10 is the microsphere-induced cerebral embolism + HGF 10 µg administered group, and ME + HGF30 is the microsphere-induced cerebral embolism + HGF 30 µg administered group, and "means ± standard error" is shown for each value, and as a statistic inspection method, two-way ANOVA was used for comparison among the groups. When significant difference by ANOVA was detected, Fisher's PLSD multiple comparison method was used as post-hoc test for further comparison among other groups. * indicates that relative to Sham, there is a significant difference (p < 0.05) with a risk factor of 5%, and # indicates that relative to ME, there is a significant difference (p < 0.05) with a risk factor of 5%. This applies to Tables 2 and 3 below.

**Table 1**

| | | Sham (n = 6) | ME (n = 8) | ME+HGF10 (n = 8) | ME+HGF30 (n = 8) |
|---|---|---|---|---|---|
| Session (on day 12 after operatio n) | 1 trial | 138.7 ± 16.1 | 145.1 ± 17.9 | 149.6 ± 18.7 | 179.2 ± 0.8 |
| | 2 trial | 91.7 ± 22.8 | 147.2 ± 20.4 | 156.9 ± 15.9 | 122.1 ± 18.3 |
| | 3 trial | 36.7 ± 7.4 | 146.8 ± 21.2* | 75.9 ± 22.5# | 98.6 ± 21.2 |
| Session (on day 13 after operatio n) | 1 trial | 72.4 ± 24.6 | 170.7 ± 6.9* | 118.7 ± 27.9 | 107.5 ± 18.6 |
| | 2 trial | 23.1 ± 5.4 | 143.4 ± 18.5* | 97.5 ± 23.3 | 45.2 ± 20.4# |
| | 3 trial | 25.6 ± 5.2 | 147.4 ± 18.4* | 68.2 ± 18.7# | 47.4 ± 14.6# |
| Session (on day 14 after operatio n) | 1 trial | 46.9 ± 18.4 | 159.5 ±18.1* | 119.5 ± 21.0 | 46.3 ± 11.1# |
| | 2 trial | 23.2 ± 7.4 | 133.2 ± 20.8* | 81.1 ± 25.6 | 29.5 ± 8.8# |
| | 3 trial | 19.6 ± 8.2 | 138.7 ± 18.7* | 71.4 ± 19.4# | 52.7 ± 13.0# |

Table 2 shows the results of the retention test on day 21 after the operation. In the sham operation group, the memory acquired in the test of the spatial memory and learning function which was carried out on days 12 to 14 after the operation was kept even on day 21 after the operation. On the other hand, in the microsphere-induced cerebral embolism group, memory retention was not completely improved. On the other hand, in the cerebral microsphere embolism + HGF 10 µg administered group, memory keeping was not recognized in the first trial, but in the second and third trials, the escape latency was significantly reduced. In the microsphere-induced cerebral embolism + HGF 30 µg administered group, the memory acquired in the test of the spatial memory and learning function which was carried out on days 12 to 14 after the operation was retained since the first trial, and as the number of trials was increased, the escape latency was shortened, and the memory and learning function was improved.

**Table 2**

| | Number of experiments | Session (on day 21 after operation) | | |
|---|---|---|---|---|
| | | 1 trial | 2 trial | 3 trial |
| Sham | 6 | 27.9 ± 7.3 | 12.8 ± 3.6 | 8.5 ± 2.5 |
| ME | 8 | 121.8 ± 19.5* | 115.5 ± 24.2* | 142.9 ± 18.8* |
| ME+HGF10 | 8 | 140.6 ± 20.9 | 53.2 ± 17.5 | 53.1 ± 19.3# |
| ME+HGF30 | 8 | 53.1 ± 18.7# | 46.4 ± 11.0# | 22.5 ± 4.0# |

Table 3 shows the results of the retention test on day 28 after the operation. In the trial on day 28, the microsphere-induced cerebral embolism + HGF 30 µg administered group, and the sham operation group showed almost the same escape latency, while the microsphere-induced cerebral embolism group showed longer escape latency in the first trial than the microsphere-induced cerebral embolism + HGF 30 µg administered group, and did not show reduction in escape latency in the second and third trials.

**Table 3**

| | Number of experiments | Session (on day 28 after operation) | | |
|---|---|---|---|---|
| | | 1 trial | 2 trial | 3 trial |
| Sham | 6 | 17.2 ± 6.0 | 7.1 ± 1.1 | 11.6 ± 3.0 |
| ME | 8 | 101.8 ± 24.4* | 90.4 ± 26.1* | 111.8 ± 24.9* |
| ME+HGF10 | 8 | 61.0 ± 16.7 | 60.9 ± 18.8 | 49.4 ± 17.0# |
| ME+HGF30 | 8 | 28.9 ± 10.0# | 19.6 ± 3.8# | 23.0 ± 4.4# |

The above results showed that long-lasting or short-term mental disorders in memory and learning function, and memory keeping ability caused by cerebral embolism with microspheres, that is, cerebral disorder, can be improved by treatment with HGF.

### Experimental Example 2. Effect of HGF on the vascular hyperpermeability caused by cerebral embolism with microspheres

The experimental animal, sample and operation of microspheres-induced cerebral embolism are the same as in Experimental Example 1.

### (1) Observation of perfused cerebral blood vessels

For the purpose of observing cerebrovascular morphology, albumin fluorescein isothiocyanate (FITC-albumin, Sigma) was injected into cerebral blood vessels. Injection of FITC-albumin into cerebral blood vessels was carried out according to the method described previously (Brain Res., 910, 81-93 (2001)) with some modification. Rats subjected to the operation of cerebral embolism with microspheres were anesthetized intraperitoneally with 40 mg/kg sodium pentobarbital (Nembutal, Abbott Laboratories) and then fixed in a supine position. After midline incision, 10 mg/mL FITC-albumin dissolved in 0.1 M PBS was injected through common carotid arteries in both sides in an amount of 1 mL per kg of the body weight at a rate of 1 mL/min. with a syringe pump (CFV-2100, Nippon Kohden Corp., Tokyo, Japan) via a polyethylene tube (SP-31, Natume, Tokyo, Japan) connected to a 2.5-mL syringe. Just after injection, the rat was hematized from the main vein of the abdomen.

For the purpose of observing the distribution of the blood vessels, FITC-albumin was injected into cerebral blood vessels to prepare a frozen coronary block using an OCT compound which is a water-soluble embedding agent, and then a thin cut section of 40 µm was prepared with a cryostat. The section was embedded in a slide glass, and an FITC green fluorescent image was observed under a fluorescence microscope.

### (2) Results

Figure 1 shows the result of image analysis of the cerebral coronary section (40 µm) on day 7 of the microsphere-induced cerebral embolism. By the cerebral embolism with microspheres, FITC-albumin leakage was observed in about 28% of the brain hemisphere. In the cerebral microsphere embolism + HGF 30 µg administered group, this leakage was completely inhibited. This means that the disruption of the brain-blood barrier is significantly ameliorated by HGF, and HGF has a protective action on vascular tissues against ischemic damage.

This also suggests that HGF inhibits acceleration of cerebrovascular permeation in various brain diseases or acceleration of vascular permeation in tissues other than the brain.

In the figure, Sham is the sham operation group, ME is the cerebral microsphere embolism group, ME + HGF is the cerebral microsphere embolism + 30 µg HGF administered group, and each column shows means ± standard error, and as a statistical test, two-way analysis of variance, i.e. two-way ANOVA was used for comparison among the groups. When significant difference by ANOVA was detected, Fisher's PLSD multiple comparison method was used as post-hoc test for further comparison among other groups. The symbol * indicates that there is a significant difference (p < 0.05) with a risk factor of 5% relative to the Sham group and # indicates that there is a significant difference (p < 0.05) with a risk factor of 5% relative to ME.

### [Formulation Example 1]

HGF (1 mg) is dissolved aseptically in 100 ml physiological saline, subdivided into a vial (1 ml/vial) with a pippete, lyophilized in a usual manner and sealed to give a lyophilized preparation.

### [Formulation Example 2]

HGF (1 mg) is dissolved aseptically in 100 ml of 0.02 M phosphate buffer (0.15 M NaCl, 0.01 w/v% polysolvate 80, pH 7.4), subdivided into a vial (1 ml/vial) with a pippete, lyophilized in a usual manner and sealed to give a lyophilized preparation.

### Industrial Applicability

The agent for improving mental disorders according to the present invention is useful in improving mental disorders, particularly decline in learning and memory function, due to cerebral dysfunction occurred in association with blood circulation disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.) and neurodegenerative disorders (for example, multiple sclerosis, Parkinson's disease, Parkinson's syndrome, Huntington's chorea, cerebrovascular dementia and Alzheimer dementia), epilepsy, head injury, etc. The agent for inhibiting hyperpermeability according to the present invention is useful as an inhibitor of vascular hyperpermeability in the brain due to blood circulation disorders in the brain (for example, brain infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema, etc.), blood leakage, cerebral edema, subcutaneous hemorrhage and cleeding tendency due to vascular hyperpermeability in various tissues (including internal organs).

The agent for inhibiting vascular hyperpermeability according to the present invention can also suppress the disruption of the blood-brain barrier, etc., and thus can prevent unnecessary substances and toxic substances from being transferred into the brain, and is also useful as an inhibitor of brain tumors and senile plaques (plaques generated by deposition of β-amyloid proteins in a part of the brain) induced by the toxic substances.

### SUMMARY

The present invention provides an agent for improving mental disorders due to cerebral dysfunction and an agent for inhibiting vascular hyperpermeability each containing a hepatocyte growth factor. The agent for improving mental disorders according to the present invention is useful in improving mental disorders, particularly decline in learning and memory function, due to cerebral dysfunction occurred in blood circulation disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema etc.) and neurodegenerative disorders (for example, multiple sclerosis, Parkinson's disease, Parkinson's syndrome, Huntington's chorea, cerebrovascular dementia and Alzheimer dementia), epilepsy, head injury, etc. The agent for inhibiting vascular hyperpermeability according to the present invention is efficacious to blood hyperpermeability in the brain due to blood circulation disorders in the brain (for example, cerebral infarction, cerebral hemorrhage, lacunar stroke, Biswanger's disease, cerebral thrombosis, subarachnoid hemorrhage, cerebrovascular moyamoya disease, carotid cerebral arterial fibrous muscular plasia, cerebral arterial sclerosis, internal carotid artery occlusion, hypertensive encephalopathy, cerebral edema etc.), blood leakage, edema, subcutaneous hemorrhage and bleeding tendency due to vascular hyperpermeability in various tissues (including internal organs).

## Claims

1. An agent for improving a mental disorder due to cerebral dysfunction, comprising a hepatocyte growth factor.

2. The agent according to claim 1, wherein the mental disorder is a decline in learning function.

3. The agent according to claim 1, wherein the mental disorder is a decline in memory function.

4. The agent according to claim 1, wherein the mental disorder is dementia.

5. An agent for inhibiting vascular hyperpermeability, comprising a hepatocyte growth factor.

6. The agent according to claim 5, wherein the vascular hyperpermeability is cerebrovascular hyperpermeability.

7. A composition for improving a mental disorder due to cerebral dysfunction, comprising a hepatocyte growth factor and a pharmaceutically acceptable additive.

8. The composition according to claim 7, wherein the mental disorder is a decline in learning function.

9. The composition according to claim 7, wherein the mental disorder is a decline in memory function.

10. The composition according to claim 7, wherein the mental disorder is dementia.

11. A composition for inhibiting vascular hyperpermeability, comprising a hepatocyte growth factor and a pharmaceutically acceptable additive.

12. The composition according to claim 11, wherein the vascular hyperpermeability is cerebrovascular hyperpermeability.

13. A method of improving a mental disorder due to cerebral dysfunction, which comprises administering a hepatocyte growth factor to a mammal.

14. The method according to claim 13, wherein the mental disorder is a decline in learning function.

15. The method according to claim 13, wherein the mental disorder is a decline in memory function.

16. The method according to claim 13, wherein the mental disorder is dementia.

17. A method of inhibiting vascular hyperpermeability, which comprises administering a hepatocyte growth factor to a mammal.

18. The method according to claim 17, wherein the vascular hyperpermeability is cerebrovascular hyperpermeability.

19. Use of a hepatocyte growth factor in producing a pharmaceutical preparation for inhibiting a mental disorder due to cerebral dysfunction.

20. The use according to claim 19, wherein the mental disorder is a decline in learning function.

21. The use according to claim 19, wherein the mental disorder is a decline in memory function.

22. The use according to claim 19, wherein the mental disorder is dementia.

23. Use of a hepatocyte growth factor in producing a pharmaceutical preparation for inhibiting vascular hyperpermeability.

24. The use according to claim 23, wherein the vascular hyperpermeability is cerebrovascular hyperpermeability.
